(19) Europäisches Patentamt
European Patent Office
Office européen des brevets

(11) **EP 2 933 330 B1**

(12) **EUROPEAN PATENT SPECIFICATION**

(45) Date of publication and mention
of the grant of the patent:
**26.12.2018   Bulletin 2018/52**

(21) Application number: **14853156.9**

(22) Date of filing: **26.02.2014**

(51) Int Cl.:
*C12N 9/20* (2006.01)        *C12N 1/00* (2006.01)
*C12N 15/63* (2006.01)       *C12N 1/19* (2006.01)
*A21D 8/04* (2006.01)

(86) International application number:
**PCT/CN2014/072549**

(87) International publication number:
**WO 2015/127596 (03.09.2015 Gazette 2015/35)**

(54) **NEW BIFUNCTIONAL LIPASE MUTANT AND USES THEREOF IN FLOUR PRODUCT PROCESSING**

NEUARTIGE DOPPELFUNKTIONS-LIPASEVARIANTE UND DEREN ANWENDUNG IN DER VERARBEITUNG VON MEHLPRODUKTEN

NOUVEAU MUTANT BIFONCTIONNEL DE LIPASE ET UTILISATIONS CORRESPONDANTES DANS LE TRAITEMENT DE PRODUITS À BASE DE FARINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.10.2015   Bulletin 2015/43**

(73) Proprietor: **Jiangnan University Wuxi, Jiangsu 214122 (CN)**

(72) Inventors:
• **XU, Yan
Wuxi
Jiangsu 214122 (CN)**
• **YU, Xiaowei
Wuxi City,
Jiangsu Province  214122 (CN)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
**CN-A- 101 974 499     CN-B- 102 653 743**

• **WANG, RUI ET AL.: 'Increasing Activity of Rhizopus chinensis CCTCC M201021 lipase by directed evolution-error prone PCR' CHINESE JOURNAL OF BIOTECHNOLOGY vol. 25, no. 12, 25 December 2009, XP055258943**

EP 2 933 330 B1

**Description**

**BACKGROUND OF THE INVENTION**

**Field of the Invention**

The present invention relates to the field of enzyme engineering. In particular, the invention relates to a novel dual-function lipase variant and its use in processing of flour products. More particularly, the invention relates to a lipase variant which was obtained by molecular biology techniques and turned out to be more suitable for processing of flour products.

**Background of the Invention**

Lipase preparation plays an irreplaceable role in improving baking and flour quality due to its unique biological characteristics. At present, adding lipase preparation during processing of flour products has become a mainstream trend .

Glycerol phospholipids and triglycerides are contained in the flour used for bread baking and the former can be partially hydrolyzed into hemolysis glycerophospholipids while the latter can be partially hydrolyzed into monoglycerides or diglycerides. These three hydrolysates would work as surfactants during emulsion reaction and improve the strength of gluten, enhance urgent expansion into the furnace and expand the volume, which make the bread uniform, soft and tasty. Therefore, addition of enzymes which possess the activity of hydrolyzing glycerol phospholipids and triglycerides during the bread baking process will help to get better products as described above. What's more, the fat-soluble pigments such as as lutein and carotene, which affect the whiteness of flour products, would be released when the enzyme is added to the flour. On this account, pigments become more easily oxidized and fade. As a result, the flour products would be whitened.

In previous studies, the inventors successfully screened *Rhizopus chinensis* CCTCC M 201021 from Luzhou-flavor liquor brewing koji, which has a high yield of lipase. And then the lipase gene of the genome was cloned for the first time and a recombinant *Pichia pastoris* with high level secretion for the lipase was obtained (Yu Xiao-Wei et al J Mol Catal B: Enzym, 2009, 57: 304-311). It has been disclosed in the granted patent CN 102160562 that lipase from *R. chinensis* CCTCC M 201021 was good at improving the baking characteristics of bread baking as an additive. As we know processing of flour products, like baking, is always carried out under high temperature. Thus, a series of lipase variants with improved thermostability were obtained by directed evolution. (Granted patent: CN 101974499; Yu Xiao-Wei et al. Microbial Cell Factories, 2012, 11:102-112). In this invention, heat resistant lipase variants with hydrolytic activity of triglyceride and lecithin were obtained by mutating the gene encoding lipase from *R. Chinensis* through molecular techniques. The lipase variants we obtained possess notable properties of increasing the specific volume as well as whitening, making themselves highly promising candidates for applications in processing of flour products.

**Detailed Description**

The goal of the present invention is to provide a novel lipase variant, which comprises an amino acid sequence with one of the following sets of substitutions compared to SEQ ID NO. 1:

mutant 1: P298T;
mutant 2: P298T/H317P;
mutant 3: P298T/H317P/V326S;
mutant 4: P298T/T218S/S234F;
mutant 5: P298T/H317P/P168L/A129S;
mutant 6: P298T/S234F/K161R/V326S;

Nomenclature for amino acid modifications in the present invention is explained in detail as follows. The mutated amino acid in the variant is marked as "original amino acid, position, substituted amino acids". For example, S234F indicates a substitution of Serine in position 234 with Phenylalanine. And the position number corresponds to the amino acid sequence of the parent lipase shown in SEQ ID NO.1. Furthermore, L180H/T128S indicates the position of 180 and 128 are substituted with Histidine and Serine respectively.

The lipase variant exhibits improved thermostability with a half-life prolonged by 14-38 fold at 40 °C compared to the parent.

Further aspects of the present invention relate to a recombinant strain harboring a sequence encoding the lipase variant and its construction. The nucleotide sequence encoding such variants is also provided by the present invention and the recombinant strain may be genetically engineered bacteria or transgenic cells harboring the DNA. In one embodiment, the recombinant strain is recombinant *P. pastoris* GS115, *P. pastoris* KM71 or *P. pastoris* SMD1168. In a preferred aspect, the strain is recombinant *P. pastoris* GS115.

Still further aspects of the present invention relate to a method of constructing the recombinant strain. The strain expressing the lipase variant is a recombinant *P. pastoris* GS115 which contains a recombinant expression vector constructed from expression vector pPIC9, pPIC3K, pPIC9K, pAO815 or pPICZ$\alpha$. In a preferred aspect, the expression vector is pPIC9.

Use of the above lipase variants for processing of flour products such as bread, steamed bread or noodles is also provided by the present invention.

The lipase variants provided by the present invention are thermostable and obtain hydrolysis activity of triglyceride and lecithin. The properties of significantly increasing the specific volume in bread baking and whitening the flour products make the variants highly promising candidates for applications in processing of flour products.

## EXAMPLES

### Media:

LB liquid medium: 10 g/l trypton, 5 g/l yeast extract, 10 g/l NaCl, pH 7.0.

MD (Minimal dextrose medium): 13.4 g/l YNB, $4\times10^{-4}$ g/l biotin, 20 g/l dextrose, 20 g/l agar.

BMGY(Buffered glycerol-complex medium): 10 g/l yeast extract, 20 g/l trypton, 13.4 g/l YNB, $4\times10^{-4}$ g/l biotin, 10 g/l glycerol, pH 6.0, 100 mM potassium phosphate.

BMMY(Buffered methanol-complex medium): 10 g/l yeast extract, 20 g/l trypton, 13.4 g/l YNB, $4\times10^{-4}$ g/l biotin, 5 g/l methanol, 100 mM potassium phosphate.

### Example 1 Construction of recombinant expression vector by site-directed mutagenesis

The pre-constructed plasmid pPIC9K-*pro*RCL (Wang Lele et al. Cloning and expression of pro- and mature Rhizopus chinensis lipase in Pichia pastoris. the high-tech communications, (2009), 19 (10): 105) contains the lipase gene of the parent strain *R. chinensis* CCTCC M201021 and it was used as a template. Site directed mutagenesis kit (QuikChange II XL Site-Directed Mutagenesis Kit, Agilent) was used to construct the following six mutated plasmids:

mutant 1: P298T,
mutant 2: P298T/H317P,
mutant 3: P298T/H317P/V326S,
mutant 4: P298T/T218S/S234F,
mutant 5: P298T/H317P/P168L/A129S,
mutant 6: P298T/S234F/K161R/V326S.

### Example 2 Construction of recombinant strain expressing the lipase variant

The mutated plasmid was transformed into competent cells by heat shock. The plasmids were extracted from the positive candidates screened from LB medium containing ampicillin and sequenced for verification.

The correct mutated plasmid was digested with restriction endonuclease *Sal*I and the products were collected and condensed. Then, the linearised plasmid was mixed with 80 $\mu$l competent *P. pastoris* GS 115 and moved to a 0.2 cm cuvette for electric shock. The said electric shock was conducted at a voltage of 1500 V with the capacitance of 25 $\mu$F and the resistance of 200 $\Omega$.

After the shock, 1 ml 1 mol/l ice-cold sorbitol solution was immediately added and mixed. Then the mixture was kept stable at 30 °C for 1 h before applying on MD plate to screen the correct recombinant which had integrated the target gene to its chromosome. In addition, the extracted genome of the recombinant was amplified by primers 5'AOX and 3'AOX for PCR identification. Furthermore, the strain containing the empty vector was used as a negative control to verify that the target gene had been integrated into the genome.

### Example 3 Expression and secretion of the lipase variant and its separation and purification

The positive recombinant was cultivated in 25 - 50 ml of BMGY medium in 250 ml glass flasks. When cultures reached an OD of 2.0 - 6.0, the cells were centrifuged and resuspended with 25 - 50 ml of BMMY medium shaken at 20 - 30 °C and 100 - 250 rpm for 72 - 144 h. Methanol was added in an amount of 1 % of the broth volume every 24 h.

Protein separation and purification was achieved by the following steps.

(1) Concentrated by 10 kDa ultrafiltration

100 ml broth was centrifuged at 4 °C and 4000 rpm for 20 min. Then the supernatant was filtered by 0.22 $\mu$m microporous membrane and concentrated to about 10 ml through an 10 kDa ultrafiltration. Later, the concentrated enzyme solution was interchanged overnight with 0.02 mol/l HAc-NaAc buffer (pH 5.0) at 4 °C.

(2) Purified by strong cation exchange chromatography

The interchanged solution was loaded onto a pre-equilibrated SP-Sepharose FF column (1.6 cm$\times$20 cm) and eluted with the same buffer (0.02 mol/l pH 5.0 HAc-NaAc buffer) to remove the unadsorbed protein. Then the absorbed protein was eluted with 0 - 0.5 M NaCl in the same buffer at a flow rate of 1 ml/min and the lipase activity component was concentrated stepwise. After that, the component was interchanged overnight with 0.05 M potassium phosphate buffer (pH 7.5) containing 1.6 M ammonium sulfate at 4 °C for use.

(3) Further purified by hydrophobic interaction chromatography

The interchanged enzyme solution of step 2 was then treated with a Phenyl-sepharose 6 FF column (1.6 cm $\times$ 20 cm). 0.05 M potassium phosphate buffer (pH 7.5) containing 1.6 M ammonium sulfate was used as the equilibration buffer and also used to elute the unadsorbed protein. Lipase was then eluted by an ammonium sulfate concentration gradient of 0.4 M in 0.05 M potassium phosphate buffer (pH 7.5) and then water at a flow rate of 0.8 ml/min. Finally, 4 ml fractions containing the active component were collected, interchanged and then freeze-dried for use.

**Example 4 Lipase properties**

(1) Hydrolysis activity

(a) Determination of triglyceride hydrolysis activity

10 ml water was added to 200 mg olive oil and 100 mg gum arabic. The mixture was then emulsified by stirring at 10000 rpm for 5 min. Next, The 200 $\mu$l emulsified solution, 100 $\mu$l 0.2 M MOPS buffer (pH 6) and 20 $\mu$l 0.1 M calcium chloride solution was mixed and held at 40 °C for 5 min. Afterwards, 40 $\mu$l enzyme solution was added, mixed and incutated at 40 °C for 5 min before terminating the enzymatic reaction with 40 $\mu$l hydrochloric acid (1 N). Finally, 400 $\mu$l 4 % Triton X-100 was added to the mixture to release the free fatty acids.

(b) Determination of lecithin hydrolysis activity

In this case, lecithin was used as a substrate. The substrate was prepared by mixing 10 ml water, 200 mg lecithin and 400 $\mu$l Triton X-100 and emulsified by stirring at 10000 rpm for 5 min. Next, the 500 $\mu$l emulsified solution, 250 $\mu$l 0.2 M MOPS buffer (pH 6) and 20 $\mu$l 0.1 M calcium chloride solution was mixed and incubated at 40 °C for 5 min. And then 40 $\mu$l enzyme solution was added, mixed and held at 40 °C for 10 min before terminating the enzymatic reaction by addition of 100 $\mu$l hydrochloric acid (1 N). Finally, 400$\mu$l 4 % Triton X-100 was added to the mixtue to release the free fatty acids.

The quantifation of the free fatty acids was carried out by adding 3 ml free fatty acid quantitative reagent NEFA to the above reaction mixture and then keeping at 40 °C for 10 min.

One unit of enzyme activity was defined as the amount of enzyme needed to release 1 $\mu$mol fatty acids per minute at 40 °C and pH 6.0.

The results are shown in Table 1. The ratio of the triglyceride hydrolysis activity and lecithin hydrolysis activity of the purified lipase variants were decreased from 4.9 to 1.5-3.2, indicating increased lecithin hydrolysis activity relative to triglyceride hydrolysis activity of the variants.

(2) Thermal stability

The determination of half-life of the enzyme at 40 °C was carried out by the following steps. The enzyme solution was treated in 40 °C at various time intervals and the percentage (%) of residual triglyceride hydrolysis activity was determined. The time (min) was set as the X-axis and the logarithm of the percentage (%) of residual activity was set as the Y-axis. The inactivation constant kinact $k_{inact}$ was determined by the slope and the half-life of the enzyme ($t_{50}$) at 40 °C was determined by $t_{50} = \ln2/k_{inact}$.

Results of thermal stability (Table 1) showed that the half-life of the variants at 40 °C were prolonged by 14-38 fold compared to the parent lipase. ∘

Table 1 Enzymatic properties of lipase

| Enzyme | Ratio of triglyceride hydrolysis activity and lecithin hydrolysis activity (U/mg : U/mg) | Prolonged fold of thermal stability compared to the parent lipase |
|---|---|---|
| the parent lipase | 4.9 | - |
| mutant 1 | 3.2 | 14 |
| mutant 2 | 2.7 | 25 |
| mutant 3 | 1.8 | 18 |
| mutant 4 | 2.9 | 29 |
| mutant 5 | 2.2 | 38 |
| mutant 6 | 1.5 | 27 |

**Example 5 Appilication of lipase variants in bread baking**

The bread baking experiments mainly focused on the impact of the lipase on the bread specific volume, and the results were compared between the parent lipase and the variants.

The preparation of bread was according to the AACC 10-10B method with a few modifications. The procedure was used with flour, salt (1 %, flour weight basis [fwb]), sugar (4 %, fwb), butter (4 %, fwb), yeast (1.5 %, fwb) and water (62.5 %, fwb) to produce dough samples (the blank control). Experimental dough was similarly prepared containing lipase at 500 U/kg (fwb). Dough was mixed in a mixer for 10 min, and then remained stable for 10 min. For the baking test, the dough was divided into 100 g pieces, rounded, kept stable for 10 min again and molded. The molded dough was placed in a pan and proofed at 38 °C and 85 % rh (relative humidity) for 90 min and then baked for 25 min in an oven set at 170 °C top and 210 °C bottom.

Bread loaf volume was measured 1 h after baking following a seed-displacement method. The specific volume (ml/g) was calculated from the loaf weight (g) and volume (ml).

$$\text{The specific volume (ml/g)} = \text{volume (ml)/weight (g)}.$$

$$\text{The increased specific volume (\%)} = (\text{the specific volume of test - the specific volume of blank control)/the specific volume of blank control} * 100 \%$$

Results shown in Table 2 indicated that the maximum increased specific volume of the bread adding the lipase variant was 28 %, while that of the parent lipase was 21 %. It is clear that the specific volume of bread could be significantly increased by the lipase variants, making them highly promising candidates for future use in bread baking.

Table 2 Effects of lipase on increased specific volume and whiteness

| Test samples | The increased specific volume (%) | The increased whiteness compared with the blank control |
|---|---|---|
| the parent lipase | 21 | 1.3 |
| mutant 1 | 22 | 1.6 |
| mutant 2 | 27 | 1.7 |
| mutant 3 | 23 | 2.2 |
| mutant 4 | 28 | 1.8 |
| mutant 5 | 25 | 1.9 |
| mutant 6 | 24 | 2.3 |

**Example 6 Application of lipase variants in steamed bread**

The experiments mainly focused on the effects of the lipase to the whiteness of the flour products such as steamed bread. And the results were compared between the parent lipase and the variants.

The preparation of steamed bread was carried out according to the following method. The materials were original steamed bread powder without any improver and 0.8 % yeast (Angel Yeast Co., Ltd.). The addition of lipase in test samples was 500 U/kg, while the blank control was 0 U/kg. 40 % water was added to the materials and mixed thoroughly to make dough. The dough was pressed (pressed into pieces and folded and then pressed for six times), hand-molded, proofed (in a dough proofing machine at 37.5 °C and 80 % rh) and then steamed for 20 min.

The whiteness was determined by averaging the whiteness of eight random positions of the steamed bread.

The increased whiteness of the blank control was set as 0. Results (shown in Table 2) indicated that the maximum increased whiteness of steamed bread adding lipase variants was 2.3 units, while it was 1.3 in the steamed bread adding parent lipase. Obviously, the whiteness of flour products such as steamed bread could be significantly increased by the lipase variants, indicating highly promising applications in processing of flour products.

<210> 1
<211> 389
<212> PRT
<213> Lipase

<400> 1

Met Val Ser Phe Ile Ser Ile Ser Gln Gly Val Ser Leu Cys Leu Leu
1 5 10 15

Val Ser Ser Met Met Leu Gly Ser Ser Ala Val Pro Val Ala Gly His
20 25 30

Lys Gly Ser Val Lys Ala Thr Asn Gly Thr Asp Phe Gln Leu Pro Pro
35 40 45

Leu Ile Ser Ser Arg Cys Thr Pro Pro Ser His Pro Glu Thr Thr Gly
50 55 60

Asp Pro Asp Ala Glu Ala Tyr Tyr Ile Asn Lys Ser Val Gln Trp Tyr
65 70 75 80

Gln Ala His Gly Gly Asn Tyr Thr Ala Leu Ile Lys Arg Asp Thr Glu
85 90 95

Thr Val Gly Gly Met Thr Leu Asp Leu Pro Glu Asn Pro Pro Ile
100 105 110

Pro Ala Thr Ser Thr Ala Pro Ser Ser Asp Ser Gly Glu Val Val Thr
115 120 125

Ala Thr Ala Ala Gln Ile Lys Glu Leu Thr Asn Tyr Ala Gly Val Ala
130 135 140

Ala Thr Ala Tyr Cys Arg Ser Val Val Pro Gly Thr Lys Trp Asp Cys
145 150 155 160

Lys Gln Cys Leu Lys Tyr Val Pro Asp Gly Lys Leu Ile Lys Thr Phe
165 170 175

Thr Ser Leu Leu Thr Asp Thr Asn Gly Phe Ile Leu Arg Ser Asp Ala
180 185 190

Gln Lys Thr Ile Tyr Val Thr Phe Arg Gly Thr Asn Ser Phe Arg Ser
195 200 205

Ala Ile Thr Asp Met Val Phe Thr Phe Thr Asp Tyr Ser Pro Val Lys
210 215 220

Gly Ala Lys Val His Ala Gly Phe Leu Ser Ser Tyr Asn Gln Val Val
225 230 235 240

```
Lys Asp Tyr Phe Pro Val Val Gln Asp Gln Leu Thr Ala Tyr Pro Asp
            245             250                 255

Tyr Lys Val Ile Val Thr Gly His Ser Leu Gly Gly Ala Gln Ala Leu
            260             265                 270

Leu Ala Gly Met Asp Leu Tyr Gln Arg Glu Lys Arg Leu Ser Pro Lys
            275             280             285

Asn Leu Ser Ile Tyr Thr Val Gly Cys Pro Arg Val Gly Asn Asn Ala
    290             295             300

Phe Ala Tyr Tyr Val Asp Ser Thr Gly Ile Pro Phe His Arg Thr Val
305             310             315             320

His Lys Arg Asp Ile Val Pro His Val Pro Pro Gln Ala Phe Gly Tyr
            325             330             335

Leu His Pro Gly Val Glu Ser Trp Ile Lys Glu Asp Pro Ala Asp Val
            340             345             350

Gln Ile Cys Thr Ser Asn Ile Glu Thr Lys Gln Cys Ser Asn Ser Ile
    355             360             365

Val Pro Phe Thr Ser Ile Ala Asp His Leu Thr Tyr Phe Gly Ile Asn
    370             375             380

Glu Gly Ser Cys Leu
385
```

SEQUENCE LISTING

<110> Jiangnan University

<120> A Novel Dual-function Lipase Variant and Its Application in Flour Products Processing

<130> 151016ep

<140> PCT/CN2014/072549
<141> 2014-02-26

<160> 1

<170> PatentIn version 3.3

<210> 1
<211> 389
<212> PRT
<213> Rhizopus chinensis

<220>

<223> Lipase

<400> 1

```
Met Val Ser Phe Ile Ser Ile Ser Gln Gly Val Ser Leu Cys Leu Leu
1               5               10              15

Val Ser Ser Met Met Leu Gly Ser Ser Ala Val Pro Val Ala Gly His
            20              25              30

Lys Gly Ser Val Lys Ala Thr Asn Gly Thr Asp Phe Gln Leu Pro Pro
        35              40              45

Leu Ile Ser Ser Arg Cys Thr Pro Pro Ser His Pro Glu Thr Thr Gly
        50              55              60

Asp Pro Asp Ala Glu Ala Tyr Tyr Ile Asn Lys Ser Val Gln Trp Tyr
65              70              75              80

Gln Ala His Gly Gly Asn Tyr Thr Ala Leu Ile Lys Arg Asp Thr Glu
                85              90              95

Thr Val Gly Gly Met Thr Leu Asp Leu Pro Glu Asn Pro Pro Pro Ile
            100             105             110

Pro Ala Thr Ser Thr Ala Pro Ser Ser Asp Ser Gly Glu Val Val Thr
        115             120             125

Ala Thr Ala Ala Gln Ile Lys Glu Leu Thr Asn Tyr Ala Gly Val Ala
        130             135             140

Ala Thr Ala Tyr Cys Arg Ser Val Val Pro Gly Thr Lys Trp Asp Cys
145             150             155             160

Lys Gln Cys Leu Lys Tyr Val Pro Asp Gly Lys Leu Ile Lys Thr Phe
            165             170             175

Thr Ser Leu Leu Thr Asp Thr Asn Gly Phe Ile Leu Arg Ser Asp Ala
            180             185             190

Gln Lys Thr Ile Tyr Val Thr Phe Arg Gly Thr Asn Ser Phe Arg Ser
        195             200             205
```

```
Ala Ile Thr Asp Met Val Phe Thr Phe Thr Asp Tyr Ser Pro Val Lys
    210             215             220

Gly Ala Lys Val His Ala Gly Phe Leu Ser Ser Tyr Asn Gln Val Val
225             230             235                 240

Lys Asp Tyr Phe Pro Val Val Gln Asp Gln Leu Thr Ala Tyr Pro Asp
                245             250                 255

Tyr Lys Val Ile Val Thr Gly His Ser Leu Gly Gly Ala Gln Ala Leu
                260             265             270

Leu Ala Gly Met Asp Leu Tyr Gln Arg Glu Lys Arg Leu Ser Pro Lys
            275             280             285

Asn Leu Ser Ile Tyr Thr Val Gly Cys Pro Arg Val Gly Asn Asn Ala
    290             295             300

Phe Ala Tyr Tyr Val Asp Ser Thr Gly Ile Pro Phe His Arg Thr Val
305             310             315                 320

His Lys Arg Asp Ile Val Pro His Val Pro Pro Gln Ala Phe Gly Tyr
                325             330             335

Leu His Pro Gly Val Glu Ser Trp Ile Lys Glu Asp Pro Ala Asp Val
            340             345             350

Gln Ile Cys Thr Ser Asn Ile Glu Thr Lys Gln Cys Ser Asn Ser Ile
        355             360             365

Val Pro Phe Thr Ser Ile Ala Asp His Leu Thr Tyr Phe Gly Ile Asn
    370             375             380

Glu Gly Ser Cys Leu
385
```

**Claims**

1.  A lipase comprising an amino acid sequence which has one of the following sets of substitutions compared to SEQ ID NO.1: P298T, P298T/H317P, P298T/H317P/V326S, P298T/T218S/S234F, P298T/H317P/P168L/A129S, or P298T/S234F/K161R/V326S.

2.  The lipase of claim 1, wherein said lipase has an amino acid sequence with substitutions of P298T/T218S/S234F compared to SEQ ID NO.1.

3.  The lipase of claim 1, wherein said lipase has an amino acid sequence with substitutions of P298T/H317P/P168L/A129S compared to SEQ ID NO.1.

4.  A nucleotide sequence encoding the lipase of anyone of claims 1 to 3.

5.  A recombinant strain harboring the nucleotide sequence of claim 4.

6.  The recombinant strain of claim 5, wherein said recombinant strain is recombinant *P. pastoris* GS115, *P. pastoris* KM71 or *P. pastoris* SMD1168.

7.  The recombinant strain of claim 5, wherein said recombinant strain is recombinant *P. pastoris* GS115.

8.  An expression vector or cloning vector harboring the nucleotide sequence of claim 4.

9. A method of constructing the recombinant strain of claim 7, wherein said method contains a recombinant expression vector and a host, the recombinant expression vector is constructed by pPIC9, pPIC3K, pPIC9K, pAO815 or pPICZa, and the host is *P. pastoris* GS115.

10. The method of claim 9, wherein the recombinant expression vector is constructed by pPIC9.

11. Use of the lipase of claim 1 for processing of flour products.

12. The use of claim 11, wherein said flour products include bread, steamed bread and noodles.

**Patentansprüche**

1. Lipase, die eine Aminosäuresequenz umfasst, welche im Vergleich zu SEQ ID Nr. 1 eine der folgenden Gruppen von Substitutionen aufweist: P298T, P298T/H317P, P298T/H317PV326S, P298T/T218S/S234F, P298T/H317P/P168L/A129S oder P298T/S234F/K161R/V326S.

2. Lipase gemäß Anspruch 1, wobei die Lipase eine Aminosäuresequenz mit den Substitutionen P298T/T218S/S234F im Vergleich zu SEQ ID Nr. 1 aufweist.

3. Lipase gemäß Anspruch 1, wobei die Lipase eine Aminosäuresequenz mit den Substitutionen P298T/H317P/P168L/A129S im Vergleich zu SEQ ID Nr. 1 aufweist.

4. Nucleotidsequenz, die die Lipase gemäß einem der Ansprüche 1 bis 3 codiert.

5. Rekombinanter Stamm, der die Nucleotidsequenz gemäß Anspruch 4 beherbergt.

6. Rekombinanter Stamm gemäß Anspruch 5, wobei es sich bei dem rekombinanten Stamm um rekombinante *P. pastoris* GS115, *P. pastoris* KM71 oder *P. pastoris* SMD1168 handelt.

7. Rekombinanter Stamm gemäß Anspruch 5, wobei es sich bei dem rekombinanten Stamm um rekombinante *P. pastoris* GS115 handelt.

8. Expressionsvektor oder Klonierungsvektor, der die Nucleotidsequenz gemäß Anspruch 4 beherbergt.

9. Verfahren zum Aufbau des rekombinanten Stamms gemäß Anspruch 7, wobei das Verfahren einen rekombinanten Expressionsvektor und einen Wirt beinhaltet, wobei der rekombinante Expressionsvektor durch pPIC9, pPIC3K, pPIC9K, pAO815 oder pPICZα aufgebaut ist und es sich bei dem Wirt um *P. pastoris* GS115 handelt.

10. Verfahren gemäß Anspruch 9, wobei der rekombinante Expressionsvektor durch pPIC9 aufgebaut ist.

11. Verwendung der Lipase gemäß Anspruch 1 zur Verarbeitung von Mehlprodukten.

12. Verwendung gemäß Anspruch 11, wobei die Mehlprodukte Brot, Dampfbrot und Nudeln umfassen.

**Revendications**

1. Lipase comprenant une séquence d'acides aminés qui comporte l'un des ensembles suivants de substitutions par rapport à la SEQ ID n° 1 : P298T, P298T/H317P, P298T/H317P/V326S, P298T/T218S/S234F, P298T/H317P/P168L/A129S, ou P298T/S234F/K161R/V326S.

2. Lipase selon la revendication 1, dans laquelle ladite lipase comporte une séquence d'acides aminés avec des substitutions de P298T/T218S/S234F par rapport à la SEQ ID n° 1.

3. Lipase selon la revendication 1, dans laquelle ladite lipase comporte une séquence d'acides aminés avec des substitutions de P298T/H317P/P168L/A129S par rapport à la SEQ ID n° 1.

**4.** Séquence nucléotidique codant pour la lipase selon l'une quelconque des revendications 1 à 3.

**5.** Souche recombinante abritant la séquence nucléotidique selon la revendication 4.

**6.** Souche recombinante selon la revendication 5, dans laquelle ladite souche recombinante est la souche recombinante *P. pastoris GS115, P. pastoris KM71* ou *P. pastoris SMD1168.*

**7.** Souche recombinante selon la revendication 5, dans laquelle ladite souche recombinante est la souche recombinante *P. pastoris GS115.*

**8.** Vecteur d'expression ou vecteur de clonage hébergeant la séquence nucléotidique selon la revendication 4.

**9.** Procédé de construction de la souche recombinante selon la revendication 7, selon lequel ledit procédé contient un vecteur d'expression recombinant et un hôte, le vecteur d'expression recombinant est construit par pPIC9, pPIC3K, pPIC9K, pAO815 ou pPICZa, et l'hôte est *P. pastoris* GS115.

**10.** Procédé selon la revendication 9, selon lequel le vecteur d'expression recombinant est construit par pPIC9.

**11.** Utilisation de la lipase selon la revendication 1 pour traiter des produits à base de farine.

**12.** Utilisation selon la revendication 11, dans laquelle lesdits produits à base de farine incluent du pain, du pain cuit à la vapeur et des nouilles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102160562
- CN 101974499
- CN 2014072549 W

**Non-patent literature cited in the description**

- **YU XIAO-WEI et al.** *J Mol Catal B: Enzym,* 2009, vol. 57, 304-311
- **YU XIAO-WEI et al.** *Microbial Cell Factories,* 2012, vol. 11, 102-112
- **WANG LELE et al.** *Cloning and expression of pro- and mature Rhizopus chinensis lipase in Pichia pastoris. the high-tech communications,* 2009, vol. 19 (10), 105